# EUROPÄISCHE PATENTSCHRIFT

(11) **EP 2 825 217 B1**
(45) Veröffentlichungstag und Bekanntmachung des Hinweises auf die Patenterteilung: **26.12.2018**
(21) Anmeldenummer: 13706009.1
(22) Anmeldetag: 26.02.2013
(51) Int. Cl.: A61L 31/04, A61L 31/16, A61L 15/32, A61L 15/46

(54) **KOLLAGENHALTIGES FLÄCHENGEBILDE**
COLLAGEN-CONTAINING SHEET MATERIAL
STRUCTURE PLANE CONTENANT DU COLLAGÈNE

(30) Priorität: 13.03.2012 DE 102012004842
(43) Veröffentlichungstag der Anmeldung: 21.01.2015
(73) Patentinhaber: OSARTIS GmbH, 65807 Dieburg (DE)
(72) Erfinder: WOLFSTÄDTER, Marco, 63939 Wörth am Main (DE); SCHÄFER, Birgit, 64832 Babenhausen (DE); SCHUBERT, Ellen, 63834 Sulzbach (DE)
(74) Vertreter: Blumbach · Zinngrebe Patent- und Rechtsanwälte
(86) Internationale Anmeldenummer: PCT/EP2013/053763
(87) Internationale Veröffentlichungsnummer: WO 2013/135477

(56) Entgegenhaltungen:
- EP-A2- 2 098 255
- US-A- 5 331 092
- US-A- 5 785 983

## Beschreibung

### Gebiet der Erfindung:

Die Erfindung betrifft ein kollagenhaltiges Flächengebilde, welches als Wundauflage, insbesondere für chirurgische Eingriffe verwendet wird.

### Hintergrund der Erfindung:

Kollagenhaltige Flächengebilde sind bekannt. So zeigt beispielsweise die Offenlegungsschrift EP 2 098 255 A2 (AAP Biomaterials GmbH) ein Verfahren zur Herstellung von Kollagenmaterial. Als Ausgangsmaterial wird Schweinehaut verwendet, welche zur Entfernung von Fett und sonstigen Fremdstoffen basisch, oxidativ und sauer aufgereinigt wird. Die Schweinehaut wird mechanisch zerkleinert und sodann wird eine wässrige Suspension aus der Schweinehaut hergestellt, welche durch Zugabe eines Phosphatpuffers auf einen neutralen bis leicht basischen pH-Wert eingestellt wird.

Es bildet sich hierdurch ein dreidimensionales Netzwerk aus den Kollagenfibrillen. Die dieses Netzwerk enthaltene Suspension wird lyophilisiert. Das so entstandene schwamm- oder vliesartige Flächengebilde ist sowohl im trockenen als auch im nassen Zustand hoch porös, sehr flexibel, und haftet nicht an dem OP-Besteck.

Nachteilig an dem Material ist für manche Anwedungen, dass es sich auf Grund seiner sehr hohen Flexibilität im rehydratisierten Zustand schlecht schneiden lässt. Weiter quillt das Material im nassen Zustand auf.

Bei einigen Anwendungen ist es besonders von Nachteil, dass das Flächengebilde opak ist. Der Operateur kann somit nicht durch das Flächengebilde hindurch schauen, was in vielen Fällen die genaue Platzierung des Flächengebildes stark erleichtern würde.

Das Dokument US 5,785,983 B zeigt das Herstellen eines Kollagenfilms aus einer Zusammensetzung, die mit einer sauren Lösung hergestellt wird.

Das Dokument US 5,331,092 B2 zeigt die Herstellung eines Kollagenschwamms.

### Aufgabe der Erfindung:

Der Erfindung liegt demgegenüber die Aufgabe zu Grunde, die genannten Nachteile des Standes der Technik zumindest zu reduzieren.

Es ist insbesondere eine Aufgabe der Erfindung, ein flexibles aber gleichzeitig im gewissen Maße transparentes Flächengebilde bereit zu stellen, bei dem zumindest im aufgelegten Zustand das darunter gelegene Gewebe zu erkennen ist.

Die Aufgabe der Erfindung wird bereits durch ein Verfahren zur Herstellung eines kollagenhaltigen Flächengebildes sowie durch ein transparentes kollagenhaltiges Flächengebilde nach einem der unabhängigen Ansprüche gelöst.

Bevorzugte Ausführungsformen und Weiterbildungen der Erfindung sind dem jeweiligen Unteransprüchen zu entnehmen.

Die Erfindung betrifft ein Verfahren zur Herstellung eines kollagenhaltigen Flächengebildes. Unter einem kollagenhaltigen Flächengebilde wird insbesondere ein Material verstanden, welches zu über 70%, vorzugsweise zu über 90% aus Kollagen besteht.

Das Herstellungsverfahren umfasst das nasschemische Aufbereiten eines Ausgangsmaterials aus Säugetierhäuten.

Vorzugsweise wird als Ausgangsmaterial Schweinehaut verwendet. Es ist aber auch denkbar auf equine oder bovine Ausgangsmaterialien zurückzugreifen.

Das basische, oxidative und saure Aufbereiten kann beispielsweise durch abwechselndes Eintauchen und Spülen in Basen, Peroxiden und Säuren erfolgen. Insbesondere kann Natronlauge, Wasserstoffperoxid und Phosphorsäure verwendet werden.

Das nasschemische Aufbereiten erfolgt um nicht erwünschtes Fremdmaterial wie Fette, Zellbestandteile und Pyrogene zu entfernen sowie um mikrobielle Organismen abzutöten.

Vorzugsweise wird das Ausgangsmaterial nach dem nasschemischen Aufbereiten sauer eingestellt.

Zum Herstellen einer kollagenhaltigen Suspension wird das Ausgangsmaterial zerkleinert. Die Zerkleinerung erfolgt vorzugsweise mechanisch, insbesondere durch Wolfen und/oder in einer Mühle nach dem Rotor-Stator-Prinzip.

Die Zerkleinerung erfolgt vorzugsweise nach dem nasschemischen Aufbereiten. Es ist aber auch denkbar das Ausgangsmaterial zuerst zu zerkleinern und dann nasschemisch aufzubereiten.

Insbesondere durch eine mechanische Zerkleinerung des Ausgangsmaterials wird eine Suspension bereitgestellt, die ein natives Kollagen enthält, bei welchem die Telopeptide größtenteils erhalten sind.

Gemäß der Erfindung wird die Suspension dann getrocknet, wobei im Unterschied zu der in der Offenlegungsschrift EP 2 098 255 A2 beschriebenen Vorgehensweise die Trocknung derart durchgeführt wird, dass sich das Kollagen während der Trocknung zumindest teilweise absetzt und eine transparente Haut bildet.

Es wird mithin kein hochporöses Material hergestellt, sondern ein dichtes folienartiges Gebilde, welches zumindest teilweise transparent ist.

Unter transparent im Sinne der Erfindung wird nicht verstanden, dass das Material im Sinne einer glasklaren Scheibe oder Folie völlig durchsichtig ist, sondern dass das Material derart transluzent ist, dass zumindest im aufgelegten Zustand das darunter liegende Gewebe erkennbar ist. Es versteht sich, dass das Material Lufteinschlüsse sowie Eintrübungen enthalten kann.

Die Trocknung kann derart durchgeführt werden, dass zunächst gewartet wird, bis sich das Kollagen überwiegend abgesetzt hat.

Gemäß der Erfindung wird die Trocknung bei Temperaturen durchgeführt werden, welche über dem Gefrierpunkt der Suspension liegen. Hierdurch sackt das Kollagenmaterial beim Trocknen zusammen und bildet ein folienartiges Flächengebilde. Es ist denkbar, die Trocknung unter Druckminderung durchzuführen.

Überraschenderweise ist das so hergestellte Flächengebilde biegbar und flexibel. Vorher war man davon ausgegangen, dass eine poröse dreidimensionale Struktur nötig ist, um ein Material bereitstellen zu können, welches nicht spröde und brüchig ist.

Durch die Erfindung konnte aber überraschenderweise ein Material bereitgestellt werden, welches transluzent und biegbar ist. Weiter lässt sich das Material im Unterschied zu einem hochporösen Fließ oder schwammartigen Material auch im rehydratisierten Zustand leicht schneiden, sodass der Anwender die Form und Größe des Materials leicht anpassen kann.

Bei einer Weiterbildung der Erfindung werden der Suspension Metallpartikel, insbesondere Silberpartikel zugesetzt.

Die Zugabe eines elementaren Metalls ermöglicht es, das Material mit weiteren Eigenschaften zu funktionalisieren. Vorzugsweise wird zur Erzielung antibakterieller Eigenschaften Silber verwendet.

Es hat sich heraus gestellt, dass das erfindungsgemäße folienartige Material Metallionen gegenüber einem porösen Material langsamer freisetzt, sodass die Wirkung länger anhält und toxische Konzentrationen direkt nach dem Einsetzten weitgehend vermieden werden können.

Die Verwendung von elementarem Silber hat gegenüber der Verwendung von Metallsalzen zudem den Vorteil, dass es allenfalls in geringem Maße zu dunklen Verfärbungen kommt.

Zur Bereitstellung einer lang anhaltenden antibakteriellen Wirkung ist oder für andere Zwecke ist auch denkbar, andere aktive Substanzen wie Bakteriostatika bzw. bakterizide Agenzien oder Antimykotika, wie beispielsweise Gentamicin oder Guanidine, zuzusetzen.

Die Metallpartikel werden vorzugsweise selbst als Suspension zugegeben.

Es können insbesondere Partikel mit einer mittleren Partikelgröße zwischen 10 nm und 10 µm verwendet werden. Vorzugsweise werden Nanopartikel verwendet, insbesondere Nanopartikel mit einer Partikelgröße von weniger als 50 nm.

Bei der Herstellung des Flächengebildes kommt es bei derartigen Nanomaterialien zwar zu Agglomerationserscheinungen, sodass das Metall in dem Flächengebilde größtenteils in Agglomeraten vorliegt. Auf die positiven Eigenschaften des erfindungsgemäßen Materials hat dies jedoch keinen Einfluss.

Das Metall wird bei einer bevorzugten Ausführungsform der Erfindung derart dosiert, dass das hergestellte Flächengebilde einen Gehalt an dem Metall von 10 bis 10.000 ppm, vorzugsweise von 100 bis 10.000 ppm, aufweist.

Gemäß der Erfindung wird der pH-Wert der Suspension vor der Trocknung auf einen Wert zwischen 5,5 und 8 eingestellt.

Insbesondere ist vorgesehen, die Suspension neutral bis leicht basisch einzustellen.

Um eine Reaktion des Silbers mit Chlor zu vermeiden, sollte die Suspension nicht mit Salzsäure angesäuert sein. Vorzugsweise wird eine im Wesentlichen chloridfreie Suspension verwendet.

Das erfindungsgemäße Material kann ohne Verwendung eines Vernetzungsmittels hergestellt werden. Die Erfindung schließt die Verwendung eines Vernetzungsmittels aber nicht aus.

Durch die Erfindung konnte weiter die Trocknung vereinfacht werden. Eine Gefriertrocknung ist nicht erforderlich, kann aber nach Absetzen des Kollagens dennoch durchgeführt werden. Vorzugsweise wird das Material unter vermindertem Druck bei einer Temperatur oberhalb des Gefrierpunktes der Suspension getrocknet.

Durch die Erfindung konnte ein zumindest teilweise transparentes kollagenhaltiges Flächengebilde bereitgestellt werden.

Dieses hat vorzugsweise eine geschlossene Porosität von weniger als 50%, besonders bevorzugt von weniger als 20%.

Das Material ist folienartig und gegenüber ähnlich stabilen fließ- oder schwammartigen Kollagenen dünner. Insbesondere hat das Flächengebilde eine Dicke zwischen 0,05 und 2,00 mm, vorzugsweise zwischen 0,1 und 1,00 mm.

Das Material kann diverse Formen annehmen, insbesondere rund, viereckig und oval.

Es versteht sich, dass in der Regel ein Sterilmaterial hergestellt wird. Die Sterilisation kann durch ionisierende Strahlung, beispielsweise mit γ- und/oder β-Bestrahlung erfolgen.

Vorzugsweise erfolgt eine Sterilisation mit Ethylenoxid, da hierdurch die Stabilität des Flächengebildes erhöht wird, wohingegen zumindest starke ionisierende Strahlung das Flächengebilde in seiner Stabilität schwächt. Möglicherweise werden durch Ethylenoxid zusätzliche Vernetzungen gebildet.

Zu Erzielung von antibakteriellen Eigenschaften wird insbesondere eine nanosilberhaltige Suspension verwendet, die einen Emulgator enthält.

Ein Verfahren zur Herstellung einer derartigen stabilen silberhaltigen Suspension ist aus der Offenlegungsschrift DE 10 2009 059 276 A1 (Rent-a-Scientist GmbH) bekannt. Auf den Offenbarungsgehalt dieses Dokumentes wird vollumfänglich verwiesen.

Das erfindungsgemäße Material hat blutstillende Eigenschaften und eignet sich aufgrund seiner antibakteriellen Eigenschaften vor allen Dingen zur Verwendung in Verbindung mit schlecht heilenden Wunden, insbesondere bei Diabetes sowie bei Brandverletzungen. Auch dentale Anwendungen sind denkbar.

### Beschreibung der Zeichnungen:

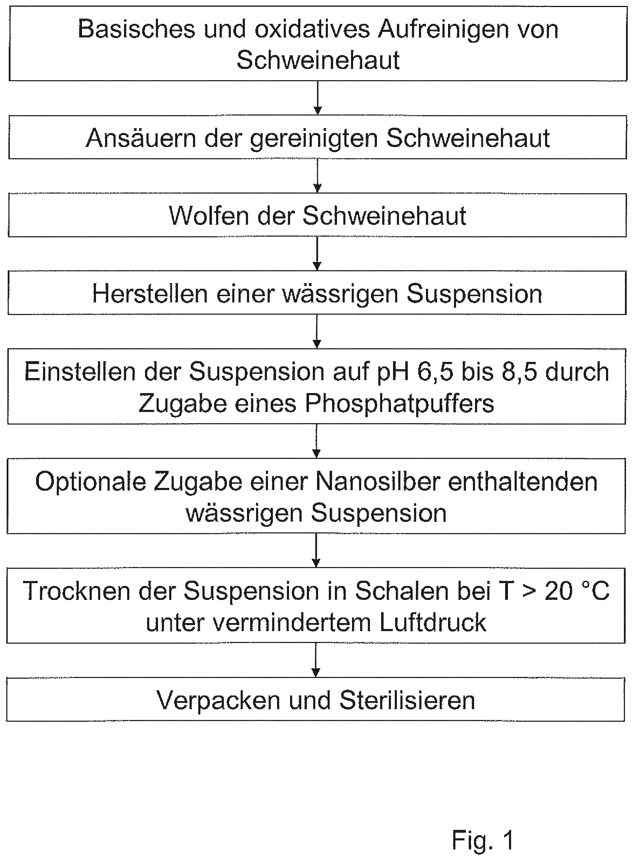

Fig. 1 zeigt ein Flussdiagramm eines Verfahrens zur Herstellung eines kollagenhaltigen Flächengebildes.

Zunächst wird Schweinehaut basisch, oxidativ und sauer aufbereitet.

Hierfür können beispielsweise Wasserstoffperoxid, Natronlauge und Phosphorsäure verwendet werden, in welche das Ausgangsmaterial abwechselnd eingetaucht und sodann ausgewaschen wird.

Durch diese nasschemische Aufbereitung wird ein Ausgangsmaterial bereit gestellt, was zu über 70%, vorzugsweise zu über 80% aus Kollagen besteht.

Sodann wird die nasschemisch aufgereinigte Schweinehaut angesäuert. Hierfür wird vorzugsweise Phosphorsäure verwendet.

Zur Herstellung einer Suspension wird die Schweinehaut zunächst durch Wolfen mechanisch zerkleinert und sodann aus dem durch weitere Zerkleinerungsschritte entstandenen Brei eine wässrige Suspension hergestellt. Diese Suspension hat insbesondere einen Feststoffgehalt zwischen 0,5 und 5%.

Sodann wird die wässrige Suspension durch Zugabe eines Phosphatpuffers auf einen neutralen bis leicht basischen pH-Wert von 6,5 bis 8,5 eingestellt.

Anschließend kann eine Nanosilber enthaltene wässrige Suspension zugesetzt werden. Es sollte dabei eine Suspension verwendet werden, die über einen längeren Zeitraum stabil ist. Das Metall wird nicht als Salz, sondern als elementares Silber zugegeben.

Um eine gute Verteilung zu erreichen, kann die Zugabe der Nanosilber enthaltenen Suspension mit Hilfe von geeigneten Dispergierhilfen, beispielsweise in einem Ultraschallbad oder einer geeigneten Mühle nach dem Rotor-Stator-Prinzip erfolgen.

Anschließend wird die Suspension in Schalen bei einer Temperatur von über 20° Celsius bei vermindertem Druck getrocknet.

Das Kollagen setzt sich dabei überwiegend als folienartiges Gebilde auf dem Schalenboden ab, wobei ein großer Teil der Silberpartikel in dem entstehenden Material festgesetzt sind.

Anschließend kann das getrocknete Flächengebilde verpackt und sterilisiert werden.

Die Sterilisation kann beispielsweise mit Ethylenoxid erfolgen.

Weiter ist eine Sterilisation des bereits verpackten Materials durch Bestrahlung möglich.

Das hergestellte Material rehydratisiert sehr schnell, hat eine gute blutstillende Wirkung und ist auch im trockenen Zustand faltbar, rollbar und leicht schneidbar.

Zudem ist das Material zumindest derart transluzent, dass das aufgelegte Material das darunter liegende Gewebe erkennen lässt. Unter dem Flächengebilde sich bildende Komplikationen wie Entzündungen können gut erkannt werden.

Weiter neigt das Material weniger zum Festkleben und kann leichter entfernt werden.

## Patentansprüche

1. Verfahren zur Herstellung eines kollagenhaltigen Flächengebildes, umfassend die Schritte:
nasschemisches Aufbereiten eines Ausgangsmaterial aus Säugetierhäuten,
- Zerkleinern des Ausgangsmaterials,
- Herstellen einer das zerkleinerte Ausgangsmaterial enthaltenden Suspension,
- Trocknen der Suspension bei einer Temperatur oberhalb des Gefrierpunktes der Suspension, wobei das Trocknen derart durchgeführt wird, dass sich das Kollagen während der Trocknung zumindest teilweise absetzt und eine transparente Haut bildet, indem das Kollagenmaterial beim Trocknen zusammensackt und ein folienartiges Flächengebilde bildet,
- Einstellen des pH-Werts der Suspension vor der Trocknung auf einen Wert zwischen 5,5 und 8.

2. Verfahren zur Herstellung eines kollagenhaltigen Flächengebildes nach dem vorstehenden Anspruch, **dadurch gekennzeichnet, dass** der Suspension Metallpartikel, insbesondere Silberpartikel, und/oder bakterizide Agenzien und/oder Antimykotika zugesetzt werden.

3. Verfahren zur Herstellung eines kollagenhaltigen Flächengebildes nach dem vorstehenden Anspruch, **dadurch gekennzeichnet, dass** Metallpartikel mit einer mittleren Partikelgröße zwischen 10 nm und 10 µm, vorzugsweise Partikel mit einer mittleren Partikelgröße von weniger als 50 nm, zugegeben werden.

4. Verfahren zur Herstellung eines kollagenhaltigen Flächengebildes nach einem der beiden vorstehenden Ansprüche, **dadurch gekennzeichnet, dass** Metallpartikel als Suspension zugegeben werden.

5. Verfahren zur Herstellung eines kollagenhaltigen Flächengebildes nach einem der vorstehenden Ansprüche, **dadurch gekennzeichnet**, die Suspension vor der Trocknung neutral bis leicht basisch eingestellt wird.

6. Transparentes kollagenhaltiges Flächengebilde, hergestellt mit einem Verfahren nach einem der vorstehenden Ansprüche.

7. Transparentes kollagenhaltiges Flächengebilde nach Anspruch 6, **dadurch gekennzeichnet, dass** das Flächengebilde eine geschlossene Porosität von weniger als 50 %, vorzugsweise von weniger als 20 % aufweist.

8. Transparentes kollagenhaltiges Flächengebilde nach einem der Ansprüche 6 oder 7, **dadurch gekennzeichnet, dass** das Flächengebilde zu über 70 %, vorzugsweise zu über 90 % aus Kollagen besteht.

9. Transparentes kollagenhaltiges Flächengebilde nach einem der Ansprüche 6 bis 8, **dadurch gekennzeichnet, dass** das Flächengebilde eine Dicke zwischen 0,05 und 2 mm, vorzugsweise zwischen 0,1 und 1 mm aufweist.

## Claims

1. A method for producing a collagen-containing sheet material, comprising the steps of:
- wet-chemical treatment of a starting material from mammalian skins;
- comminuting the starting material;
- preparing a suspension containing the comminuted starting material;
- drying the suspension at a temperature above the freezing point of the suspension, wherein the drying is performed in such a manner that at least part of the collagen settles during the drying and forms a transparent skin, as the collagen material sags together during drying and forms a film-like sheet material;
- adjusting the pH value of the suspension prior to the drying to a value between 5.5 and 8.

2. The method for producing a collagen-containing sheet material as claimed in the preceding claim, wherein metal particles, especially silver particles, and/or bactericidal agents and/or antifungal agents are added to the suspension.

3. The method for producing a collagen-containing sheet material as claimed in the preceding claim, wherein metal particles are added, which have an average particle size between 10 nm and 10 µm, preferably particles having an average particle size of less than 50 nm.

4. The method for producing a collagen-containing sheet material as claimed in any of the two preceding claims, wherein the metal particles are added as a suspension.

5. The method for producing a collagen-containing sheet material as claimed in any of the preceding claims, wherein prior to the drying the suspension is adjusted to a neutral to slightly basic value.

6. A transparent collagen-containing sheet material, produced by a method according to any of the preceding claims.

7. The transparent collagen-containing sheet material as in claim 6, wherein the sheet material has a closed porosity of less than 50 %, preferably less than 20 %.

8. The transparent collagen-containing sheet material as claimed in any of the claims 6 or 7, wherein the sheet material comprises more than 70 %, preferably more than 90 % of collagen.

9. The transparent collagen-containing sheet material according to any of the claims 6 to 8, wherein the sheet material has a thickness between 0.05 and 2 mm, preferably between 0.1 and 1 mm.

## Revendications

1. Procédé de fabrication d'une structure plane contenant du collagène, comprenant les étapes :
préparation par voie chimique humide d'un matériau de départ en peaux de mammifère,
- pilage du matériau de départ,
- fabrication d'une suspension contenant le matériau de départ pilé,
- séchage de la suspension à une température supérieure au point de congélation de la suspension, où le séchage est effectué de telle sorte que le collagène se dépose au moins partiellement pendant le séchage et forme une peau transparente par le fait que le matériau de collagène s'affaisse avec le séchage et forme une structure plane en forme de feuille,
- réglage de la valeur de pH de la suspension avant le séchage sur une valeur entre 5,5 et 8.

2. Procédé de fabrication d'une structure plane contenant du collagène selon la revendication précédente, **caractérisé en ce que** des particules métalliques, en particulier des particules d'argent, et/ou des agents bactéricides et/ou des antimycosiques sont ajoutées à la suspension.

3. Procédé de fabrication d'une structure plane contenant du collagène selon la revendication précédente, **caractérisé en ce que** des particules métalliques avec une granulométrie moyenne entre 10 nm et 10 µm, de préférence des particules avec une granulométrie moyenne de moins de 50 nm, sont ajoutées.

4. Procédé de fabrication d'une structure plane contenant du collagène selon l'une des deux revendications précédentes, **caractérisé en ce que** des particules métalliques sont ajoutées en tant que suspension.

5. Procédé de fabrication d'une structure plane contenant du collagène selon l'une des revendications précédentes, **caractérisé en ce que** la suspension est ajustée de neutre à modérément basique avant le séchage.

6. Structure plane transparente contenant du collagène, fabriquée avec un procédé selon l'une des revendications précédentes.

7. Structure plane transparente contenant du collagène selon la revendication 6, la structure plane étant **caractérisée en ce qu'**elle présente une porosité fermée de moins de 50 %, de préférence de moins de 20 %.

8. Structure plane transparente contenant du collagène selon l'une des revendications 6 ou 7, la structure plane étant **caractérisée en ce qu'**elle se compose à plus de 70 %, de préférence à plus de 90 %, de collagène.

9. Structure plane transparente contenant du collagène selon l'une des revendications 6 à 8, la structure plane étant **caractérisée en ce qu'**elle présente une épaisseur d'entre 0,05 et 2 mm, de préférence d'entre 0,1 et 1 mm.
